(19) Europäisches Patentamt
European Patent Office
Office européen des brevets

(11) Publication number: 0 133 739 B1

(12) **EUROPEAN PATENT SPECIFICATION**

(45) Date of publication of patent specification: **10.09.86**

(51) Int. Cl.$^4$: **C 07 C 31/20**, C 07 C 29/17, C 07 C 29/32, C 07 D 307/08

(21) Application number: **84303842.3**

(22) Date of filing: **06.06.84**

(54) **Process for preparing 2-alkyl-1,4-butanediols.**

(30) Priority: **08.06.83 US 502317**

(43) Date of publication of application: **06.03.85 Bulletin 85/10**

(45) Publication of the grant of the patent: **10.09.86 Bulletin 86/37**

(84) Designated Contracting States: **BE CH DE FR GB IT LI LU NL SE**

(58) References cited:
**DE-A-2 236 734**
**US-A-3 859 369**

(73) Proprietor: **E.I. DU PONT DE NEMOURS AND COMPANY**
**1007 Market Street**
**Wilmington Delaware 19898 (US)**

(72) Inventor: **Ernst, Richard Edward**
**292 Unionville Lenape Road**
**Kennett Square Pennsylvania 19348 (US)**

(74) Representative: **Woodcraft, David Charles et al**
**BROOKES & MARTIN High Holborn House 52/54 High Holborn**
**London, WC1V 6SE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 133 739 B1

**Description**

This invention relates to a process for the preparation of 2 - alkyl - 1,4 - butanediols. It is more particularly directed to a process for preparing such butanediols from 1,4 - butynediol or 1,4 - butenediol by catalytic hydrogenation and reaction with an aldehyde.

The 2 - alkyl - 1,4 - butanediols, especially 2 - methyl - 1,4 - butanediol, are a useful class of compounds in that they can be cyclized to the corresponding 3 - alkyltetrahydrofurans, which in turn can be copolymerized with tetrahydrofuran to form polyethers useful in preparing polyurethane elastomers.

In the past, these 2 - alkylbutanediols have been prepared by the reduction of itaconic acid, or by the hydroformylation and hydrogenation or 1,4 - butenediol described by Copelin in his U.S. Patent 3,859,369.

It has now been found, according to the process of this invention, that 2 - alkylbutanediols can be prepared from 1,4 - butynediol or 1,4 - butenediol by catalytic hydrogenation and reaction with an aldehyde. It is believed that the process proceeds according to the equations

(1) $HOCH_2C{\equiv}CCH_2OH + H_2 \rightarrow HOCH_2CH{=}CHCH_2OH$

(2) $HOCH_2CH{=}CHCH_2OH + 2H_2 + R\overset{O}{\overset{\|}{C}}H \longrightarrow HOCH_2\underset{CH_2R}{CH}{-}CH_2CH_2OH + H_2O$

where R is hydrogen or an alkyl radical of 1—4 carbon atoms.

When the starting material is butynediol (equation 1), intermediate butenediol is produced, which is further hydrogenated to the product 2 - alkyl - 1,4 - butanediol. The same product can also be made by using the butenediol as the starting material and omitting the step of equation 1 entirely.

In either case the reaction is conveniently conducted by bringing together, at a pH of about 9—12, and at a temperature and pressure suitable for reaction, the butynediol or butenediol, hydrogen, an appropriate aldehyde, and a suitable hydrogenation catalyst.

When butynediol is used as the starting material, the process may be run by itself, but it has been found, surprisingly, that it can also be run simultaneously with and in the same reaction vessel as the process shown in British Patent 1,242,358, according to which 1,4 - butanediol is produced from 1,4 - butynediol by catalytic hydrogenation using Raney nickel as the catalyst.

Whether the process is run by itself or not, or whether it begins with butynediol or butenediol, the process produces a mixture of 1,4-butanediol and a 2 - alkyl - 1,4 - butanediol. While these can be easily separated by conventional procedures, it may be desirable to keep the mixture intact for it can be cyclized in one step to give a mixture of tetrahydrofuran and a 3 - alkyltetrahydrofuran. A tetrahydrofuran/3 - alkyltetrahydrofuran copolymer can then be prepared directly from this mixture by adding a suitable catalyst and holding the mixture under conditions suitable for copolymerization.

The process of the invention can be run batchwise, but is more conveniently and preferably run continuously.

In the continuous mode, a column reactor of appropriate dimensions is packed in the usual manner with a conventional hydrogenation catalyst. Suitable catalysts are, for example, platinum, Raney nickel and cobalt. Raney nickel, especially Raney nickel from which about 25% by weight of aluminum has been removed, is preferred. The catalyst may be in any convenient form, but is ordinarily and preferably granular.

The butynediol used may be any commercially available type, and may be, for example, that obtained by catalytically reacting acetylene and formaldehyde, using a copper acetylide complex as the catalyst, as described in U.S. Patent 3,560,576 to Kirchner.

The butenediol, if that is used as a starting material, can likewise be of any commercially available type, and may be, for example, that obtained by the conventional hydrogenation of butynediol.

The pH value of the butynediol or butenediol is adjusted to the range 8—14, preferably about 10—11, and is then fed continuously through the reaction column.

The aldehyde, ordinarily as an aqueous solution, is also fed continuously through the column. The aldehyde will be one having the structure shown in equation (1), and will preferably be formaldehyde.

Enough aldehyde is fed into the column to provide a diol/aldehyde weight ratio of 2/1 to 200/1, preferably 10/1 to 25/1.

If the butynediol is obtained by the previously mentioned Kirchner method, it may already contain the proper amount of formaldehyde as an impurity, and separate addition of formaldehyde to the column may be unnecessary.

Hydrogen is continuously fed into the column, in co-current or countercurrent flow to the other reactants, and is conveniently maintained in the column at a pressure of 6895—55160 kPa (gauge), preferably 34475 kPa (gauge).

The exit temperature of the reaction mass conveniently is maintained at 100—200°C, preferably about 140°—150°, by recycling according to well known chemical engineering principles.

Flow of reactants into the reactor is conveniently regulated to give them a residence time in the reactor of 30—200 minutes, preferably 100—120 minutes.

The 2 - alkylbutanediol is removed from the column in liquid form, as a mixture with 1,4 - butanediol. The alkylbutandiol can be separated

from the 1,4 - butanediol, if one wishes to do this, by fractional distillation conducted according to well known chemical engineering principles.

The process of the invention, when run in the batch mode, is conducted under basically the same conditions, using the proportions of reactants and the recovery procedures just described.

Whether run batchwise or continuously, the 2 - alkylbutanediol produced may be catalytically cyclized to a corresponding 3 - alkyltetrahydrofuran, using sulfuric acid as the catalyst, by the general method disclosed in U.S. Patent 3,726,905 to Coates and Reilly. If the product, a mixture of 1,4 - butanediol and 2 - alkyl - 1,4 - butanediol, is kept intact as it comes from the reaction column, both components can be simultaneously cyclized using this method, to give a corresponding mixture of tetrahydrofuran and 3 - alkyltetrahydrofuran.

This mixture, or one like it made by mixing separate components, can then be copolymerized to form a tetrahydrofuran/3 - alkyltetrahydrofuran copolymer using fluosulfonic acid as the catalyst, as disclosed in U.S. Patent 3,358,042 to Dunlop and Sherman. This copolymer can then be used to prepare a polyurethane by the general method disclosed in U.S. Patent 4,120,850 to Pechhold.

Examples

In the following examples, all parts are by weight.

Example 1

Into a fixed-bed column reactor 76 cm long, with an inside diameter of 4.5 cm, were packed 1000 gm of Raney nickel alloy 25% of whose aluminum had been removed with caustic.

1,4 - Butynediol, a 50% aqueous solution containing 0.4% of formaldehyde, prepared as shown in U.S. Patent 3,560,576, was brought to pH 11 with sodium hydroxide and then continuously fed into the bottom of the column at a rate of 9 ml per minute.

Hydrogen was pumped into the bottom of the column and maintained in the column at a pressure of about 34475 kPa (gauge).

The exit temperature of the product was maintained at about 140°C by recycling.

The product, a 1/20 mixture of 2 - methyl - 1,4 - butanediol and 1,4 - butanediol, and also containing a small amount of butanol, was removed from the top of the column at a rate of 9 ml per minute.

Example 2

Into a shaker tube were charged

| | |
|---|---|
| 1,4-butynediol (50% solution in water) | 40 parts |
| Formaldehyde (37% solution in water) | 4 parts |
| Sodium hydroxide (50% solution in water) | 0.6 part |
| Raney nickel (slurry grade, from which substantially all aluminum had been removed) | 5 parts |

The resulting slurry had a pH of 11—12.

The slurry was heated to and held at 150°C and a hydrogen pressure of 34475 kPa (gauge), and shaken for one hour.

The product was a mixture the organic portion of which was composed of 13.2% 2 - methyl - 1,4 - butanediol, 76.7% 1,4 - butanediol, and minor amounts of methanol and butanol.

Example 3

Twenty ml of product like that produced in Example 1, and composed of 2.3% of 2 - methyl - 1,4 - butanediol, 42% of 1,4 - butanediol and the remainder water and a small amount of impurities, was distilled until the pot temperature reached 150°C. The residue was cooled to 100°C, and to it was then added 0.25 ml of concentrated sulfuric acid.

This mixture was heated to 115°C and held there for about 30 minutes, while the product, the organic portion of which contained 8.4% of 3 - methyl - tetrahydrofuran and 91.6% of tetrahydrofuran, distilled off.

Example 4

Into a shaker tube were charged

| | |
|---|---|
| 1,4-Butenediol (50% solution in water) | 40 parts |
| Formaldehyde (37% solution in water) | 2 parts |
| Sodium hydroxide (50 solution in water) | 0.6 part |
| Raney nickel (slurry grade, from which substantially all aluminum had been removed) | 5 parts |

The resulting slurry had a pH of 11.9.

The slurry was heated to and held at 140°C and a hydrogen pressure of 20,684 kPa (gauge) and shaken for 2 hours.

The product was a mixture the organic portion of which was composed of 10.4% 2 - methyl - 1,4 - butanediol, 73.5% 1,4 - butanediol and minor amounts of methanol and butanol.

**Claims**

1. A process for preparing a mixture of 1,4 - butanediol and a 2 - alkyl - 1,4 - butanediol, the process comprising bringing together, at a pH of 8—14 and a temperature and pressure suitable for reaction,

(a) hydrogen,

(b) a hydrogenation catalyst, and

(c) 1,4 - butynediol or 1,4 - butenediol, and an aldehyde of the structure:—

R—CHO

wherein R is hydrogen of an alkyl radical of 1—4 carbon atoms,

in a butynediol/aldehyde or butenediol/aldehyde weight ratio of 2/1 to 200/1.

2. A process as claimed in claim 1 in which the aldehyde is formaldehyde.

3. A process of claim 1 or claim 2, run at a pH of 9—12.

4. A process as claimed in claim 3, run at a pH of 10—11.

5. A process as claimed in any preceding claim wherein the hydrogenation catalyst is platinum, Raney nickel or cobalt.

6. A process for preparing a 2 - alkyl - 1,4 - butanediol, the process comprising (a) performing a process as claimed in any preceding claim and (b) separating the resulting 2 - alkylbutanediol product from the reaction mass.

7. A method for preparing a mixture of tetrahydrofuran and a 3 - alkyltetrahydrofuran, the method comprising catalytically cyclizing the mixture of 1,4 - butanediol and 2 - alkyl - 1,4 - butanediol produced by a process as claimed in anyone of claims 1 to 5.

8. A method for preparing a copolymer of tetrahydrofuran and a 3 - alkyltetrahydrofuran, the method comprising adding a suitable polymerization catalyst to the mixture produced by the process claimed in claim 7 and holding the mixture under conditions suitable for polymerization.

**Patentansprüche**

1. Verfahren zur Herstellung eines Gemisches von 1,4 - Butandiol und einem 2 - Alkyl - 1,4 - butandiol, das Verfahren umfassend das Inkontaktbringen, bei einem pH-Wert zwischen 8 und 14 und für die Reaktion geeigneten Temperaturen und Drücken, von

(a) Wasserstoff,

(b) einem Hydrierungskatalysator und

(c) 1,4 - Butindiol oder 1,4 - Butendiol, zusammen mit einem Aldehyd der Formel

R—CHO

in welcher Formel R für H oder für Alkyl mit 1 bis 4 C - Atomen steht und bei einem Butindiol/Aldehyd- bzw. Butendiol/Aldehyd - Gewichtverhältnis von 2:1 bis 200:1.

2. Verfahren gemäss Patentanspruch 1, in dem der Aldehyd Formaldehyd ist.

3. Verfahren gemäss Patentanspruch 1 oder 2, durchgeführt bei einem pH-Wert zwischen 9 bis 12.

4. Verfahren gemäss Patentanspruch 3, durchgeführt bei einem pH-Wert von 10 bis 11.

5. Verfahren gemäss einem der vorangehenden Patentansprüche, in dem der Hydrierungskatalysator Platin, Raney-Nickel oder Kobalt ist.

6. Verfahren zur Herstellung von 2 - Alkyl - 1,4 - butandiol, das Verfahren umfassend (a) des Ausführen eines Verfahrens gemäss irgendeinem der vorangehenden Patentansprüche und (b) das Abtrennen des erhaltenen 2 - Alkylbutandiolproduktes von der Reaktionsmasse.

7. Verfahren zur Herstellung eines Gemisches von Tetrahydrofuran und einem 3 - Alkyltetrahydrofuran, das Verfahren umfassend die katalytische Zyklisierung des Gemisches aus 1,4 - Butandiol und 2 - Alkyl - 1,4 - Butandiol, hergestellt nach einem Verfahren gemäss einem der Patentansprüche 1 bis 5.

8. Verfahren zur Herstellung eines Copolymeren von Tetrahydrofuran und von einem 3 - Alkyltetrahydrofuran, das Verfahren umfassend die Zugabe eines geeigneten Polymerisations-Katalysatoren zu einem Gemisch, welches nach dem Verfahren gemäss Patentanspruch 7 hergestellt wurde und das Halten des Gemisches unter für die Polymerisation geeigneten Bedingungen.

**Revendications**

1. Un procédé pour préparer un mélange de 1,4 - butanediol et d'un 2 - alkyl - 1,4 - butanediol, le procédé consistent à mettre en contact, à un pH de 8—14 et à une température et une pression convenant pour la réaction,

(a) de l'hydrogène,

(b) un catalyseur d'hydrogénation, et

(c) du 1,4 - butynediol ou du 1,4 - butènediol, et un aldéhyde de la structure:

R—CHO

où R est de l'hydrogène ou un radical alkyle de 1 à 4 atomes de carbone,

en un rapport en poids butynediol/aldéhyde ou butènediol/aldéhyde de 2/1 à 200/1.

2. Un procédé tel que revendiqué dans la revendication 1 dans lequel l'aldéhyde est le formaldéhyde.

3. Un procédé selon la revendication 1 ou la revendication 2, mis en oeuvre à un pH de 9—12.

4. Un procédé tel que revendiqué dans la revendication 1, mis en oeuvre à un pH de 10—11.

5. Un procédé tel que revendiqué dans l'une quelconque des revendications précédentes, dans lequel le catalyseur d'hydrogénation est le platine, le nickel de Raney et le cobalt.

6. Un procédé pour préparer un 2 - alkyl - 1,4 - butanediol, le procédé consistant (a) à conduire un procédé tel que revendiqué dans l'une quelconque des revendications précédentes et (b) à séparer de la masse réactionnelle le 2 - alkylbutanediol résultant produit.

7. Un procédé pour préparer un mélange de tétrahydrofuranne et d'un 3 - alkyltétrahydrofuranne, le procédé consistant à cycliser par voie catalytique le mélange de 1,4 - butanediol et de 2 - alkyl - 1,4 - butanediol produit par un procédé tel que revendiqué dans l'une quelconque des revendication 1 à 5.

8. Un procédé pour préparer un copolymère de

tétrahydrofuranne et d'un 3 - alkyltétrahydrofuranne, le procédé consistant à ajouter un catalyseur de polymérisation approprié au mélange produit par le procédé revendiqué dans la revendication 7 et à maintenir le mélange dans des conditions convenant à une polymérisation.